Europäisches Patentamt

**European Patent Office**

Office européen des brevets

(19)

(11) Publication number: **0 028 044**
A1

(12) # EUROPEAN PATENT APPLICATION

(21) Application number: **80200981.1**

(22) Date of filing: **16.10.80**

(51) Int. Cl.³: **C 12 N 9/58,** C 12 N 9/99,
A 23 C 19/032, A 23 C 21/00

(30) Priority: **24.10.79 EP 79200611**

(43) Date of publication of application: **06.05.81**
**Bulletin 81/18**

(84) Designated Contracting States: **AT BE CH DE FR GB IT
LI LU NL SE**

(71) Applicant: **RAPIDASE B.V., Wateringseweg 1 P.O.-box 1,
NL-2600 MA Delft (NL)**

(72) Inventor: **Colein, Bernard Albert Victor, Rue Leo
Lagrange 19, F-59263 Houplin-Ancoisne (FR)**
Inventor: **Delecourt, Robert Louis Felix, Rue Roger
Bourry 26, F-59113 Séclin (FR)**

(74) Representative: **Van der Straaten, Jan Anthony et al, c/o
GIST-BROCADES N.V. Patents and Trademarks
Department Wateringseweg 1 P.O. Box 1, NL-2600 MA
Delft (NL)**

(54) **Thermally sensitive microbial rennin and process for its preparation.**

(57) Thermally sensitive microbial rennin and process for the preparation of thermally sensitive microbial rennin, comprising the treatment of the microbial rennin containing liquid, obtained after recovery of the fermentation broth, with a modifying agent, which enhances the termal instability of microbial rennin, while retaining its milk coagulating activity at temperatures of normal cheese manufacturing, giving rise to easily decomposition of the microbial rennin in the residual liquids, obtained after cheese preparation, to be used for further food processing; as well as the use of such obtained thermally sensitive microbial rennin for the cheese preparation and the use of the obtained whey for further food processing.

EP 0 028 044 A1

0028044

## Thermally sensitive microbial rennin, a process for its preparation and its application for cheese preparation.

The invention relates to a thermally sensitive microbial rennin, to a process for the preparation of such thermally sensitive microbial rennin as well as the use of such obtained thermally sensitive microbial rennin for the cheese preparation and the use of the obtained whey for further food processing.

It is well known to prepare various types of milk coagulating enzymes, further to be mentioned microbial rennin, by cultivation of certain Mucor miehei strains in suitably selected culture mediums and recovering the microbial rennin from the fermentation broth.

Such processes are disclosed in e.g. U.S. patent no. 3988207 and U.S. patent no. 4136201.

Moreover from U.S. patent 4136201 and more particularly U.S. patent no. 3616233 it was known to prepare microbial rennin, which is substantially free of lipase and/or esterase.

The so obtained milk coagulating enzymes are successfully applied for the preparation of various types of cheese, giving rise to a major byproduct called whey.

Whey has been looked upon as a potential starting material for processes to prepare a variety of food stuffs.

Such processes essentially comprise the pasteurization and spray drying of whey in order to obtain a powder which is very rich in valuable ingredients of food stuffs. This powder is subsequently mixed with milk or liquids derived from milk in order to obtain homogeneous, attractive looking finally desired products.

A well recognized problem of this processing of the whey is the occurrence therein of residual, still active microbial rennin, which is still able to coagulate milk or liquids derived from milk and which actually prevents the preparation of the desired homogeneous, attractive looking products.

It was found that whey containing calf rennet, did not give such problems, due to a relative greater thermal instability of such rennin, and that the hereinbefore mentioned problems, connected with the use of microbial rennin containing whey, could only be eliminated by a previous heating step to destruct residual microbial rennin, using relatively high temperatures; such procedure, however, gives rise to unattractively coloured products.

This relative thermal stability of usual microbial rennin derived from Mucor miehei or Mucor pusillus Lindt may be derived from e.g. Ann.Fals.Exp.Chim., Mars 1977, 70, no. 751, pages 165-175 as referred to in Chemical Abstracts vol. 87, 51817a (1977), Biochimica et Biophysica Acta 328 (1973), 52-60, J.Dairy Science, vol. 62, no. 3 (1979-03) p. 373-377 and J.Dairy Science, vol. 62, (1979) 1227-1232.

However, it will be appreciated that not any teaching or suggestion of how to prepare a thermally sensitive microbial rennin was given in these publications.

On the other hand, from e.g. U.S. patent 4086139 was known the selective inactivation of amylase in a mixture of protease and amylase by contacting the mixture with hypochlorite or chlorite ions as oxidizing agent in an amount which is effective for the inactivation of the amylase to a substantially greater extent than the protease.

From U.S. patent no. 4087330 was known the regeneration of a spent immobilised enzyme composition by treating it with aqueous sodium hypochlorite, the spent immobilised enzyme composition comprising a porous, high surface area, insoluble inorganic support material having contaminants associated with it. All contaminants are removed by this regeneration process whereafter the support material is reacted with an enzyme, such as glucose isomerase, glucoamylase or lactase, in aqueous solution. However, this process is primarily used for the removal of the enzyme residues, which have become inactive after their use.

Moreover the Belgian patent no. 848175, discloses the chemical fixation of organic substances containing a sugar residue to a support containing at least one reactive amino group, by the oxidation of at least one hydroxymethyl group in the sugar residue to an aldehyde group, and reacting the resulting aldehyde group with amino groups carried by side chains fixed to an insoluble solid support. This process can be used to immobilise glycoproteins, such as enzymes, while as oxidizing agent e.g. a sodium iodite is used.

One of the objects of this invention is to provide a novel thermally sensitive microbial rennin which has retained the major part of its milk coagulating activity at the ordinary temperature of cheese-making.

With the term "thermally sensitive" is meant to indicate that the microbial rennin looses its milk coagulating activities almost completely at temperatures above 60$^\circ$C.

Another object is to provide a novel process for producing such a thermally sensitive microbial rennin.

A further object is to provide an improved process for the preparation of cheese, using the novel thermally sensitive microbial rennin.

An additional object is to provide an improved method for the preparation of food stuffs derived from the whey, which is the residue of the cheese preparation, using the novel, thermally sensitive microbial rennin.

The present invention relates to a novel, thermally sensitive microbial rennin, which has retained the major part of its milk coagulating activity under conditions of usual cheese manufacturing, and more specifically relates to a thermally sensitive microbial rennin, which becomes deactivated under normal process conditions for the preparation of food stuffs, derived from whey, i.e. it looses its milk coagulating activities almost completely at temperatures above 60$^\circ$C.

As a result of research and experimentation it was surprisingly found that novel thermally sensitive microbial rennins can be prepared by a process, comprising the treatment of a microbial rennin containing liquid with a chemically modifying agent.

The process may be applied as well to a filtrate, derived by recovery of the fermentation broth, obtained by cultivation of a Mucor strain in a suitable nutrient medium and preferably a Mucor miehei strain, or to a more or less

0028044

concentrated filtrate, obtained by evaporation under reduced pressure or by ultrafiltration of such filtrate.

As suitable chemically modifying agents for the presently proposed treatment of the microbial rennin,compounds such as salts of periodic acid, peracetic acid, hypochloric acid, chloric acid and iodic acid, or mixtures thereof, can be used. Preferably the sodium, potassium or calcium salts of these acids and more especially sodium hypochlorite are used.

The modifying process is preferably carried out in an aqueous solution of sodium hypochlorite, having an activity corresponding with 0.5-4 g of active chlorine per liter of starting medium and more preferably 1.5-2 g of active chlorine per liter of microbial rennin containing medium.

According to a suitable alternative embodiment as modifying agent sodium chlorite in an aqueous solution providing an amount of from 0.1-2 g of sodium chlorite per liter of microbial rennin containing medium is used.

According to a preferred embodiment of the invention, the agents are added to the microbial rennin containing liquid, in the form of 5-20 ml of an aqueous stock solution, having an activity corresponding with 140-160 g of active chlorine per liter, per liter of microbial rennin containing medium.

The obtained degree of the loss of milk coagulating activity is found to increase with increasing concentration of e.g. hypochlorite and varies in the range of from 0-15% of the original activity.

The process is carried out at temperatures varying from the temperature of freezing the solution to room temperature, i.e. at temperatures in the range of from -10 to +25$^{\circ}$C, whereby the higher temperatures in the range have been found to give rise to more inactivation, although such an increase has been found to be very weak.

The process can be carried out at pH values varying in the range of from 2 to 8 and more preferably in the range of from 2.5 to 5.

According to another feature of the invention, it was surprisingly found that as a result of the modifying reaction a thermally sensitive microbial rennin could be obtained, which was substantially free of any lipase and/or esterase activity, any of such activity, present in the microbial rennin containing starting liquid being completely deactivated by and as a result of the process.

Therefore, according to a preferred embodiment of the process of the present invention, the originally present lipase and/or esterase activity is eliminated by carrying out the process at a pH value of about 2.5 in order to eliminate the usual previous separate inactivation step for this accompanying lipase/esterase while the present process may successfully be carried out at pH values in the range of from 4-5, when starting from microbial rennin containing solutions which are substantially free of lipase and/or esterase.

The major part of the thermal instability of the desired microbial rennin can be obtained according to the process of the present invention within 1-15 minutes after mixing the modifying agent with the microbial rennin containing solution, while for obtaining the desired degree of thermal instability, total reaction times of up to 4 hours may be necessary, depending on the actual activity of the modifying agent, the working temperature and pH value.

During the process of the present invention, the coloration of the microbial rennin decreases significantly (10 to 25%), while the finally obtained microbial rennin moreover has a more attractive appearance and smell as compared to the untreated microbial rennin.

On the other hand the modified thermal sensitive microbial rennin obtained according to the process of the present invention did neither reveal any indication during further extensive investigation e.g. by infrared and mass spectiometry analysis, carried out on treated and untreated samples and comparison of the results, of the presence of halogenated organic compounds in the treated samples, nor any free chlorine could be detected.

It will be appreciated that these improved characteristics of the thermally sensitive microbial rennin represent an advantage inherent in the present invention.

Moreover, another advantage of the process of the present invention is that it can be carried out in rather simple equipment, e.g. in a usual tank with a stirrer and is consisting of rather simple measures at all.

In this connection it will be appreciated that the rate of the addition of the modifying agent has appeared to have only little influence or no influence at all on the loss of milk coagulating activity of the microbial rennin when subjected to the treatment according to the invention.

The thermally sensitive microbial rennin according to the present invention has a normal shelf life not essentially different from that of ordinary untreated microbial rennins, when stored at pH values in the range of from 4.0 to 7.0 and preferably of from 5.0 to 6.0.

According to a preferred embodiment of the process of the present invention, it has appeared to be possible to obtain a thermally sensitive microbial rennin which has lost about 5% of its original milk coagulating activity, by a treatment with 10-15 ml of a sodium hypochlorite solution, having an activity corresponding with 100-200g and more preferably about 150 g active chlorine/l, per liter of microbial rennin containing medium, i.e. fermentation liquor, at a pH between 3.7 and 5.0 and for a wide range of reaction times.

According to another preferred embodiment of the process it has appeared to be possible to prepare a thermally sensitive microbial rennin, which is substantially free of lipase and/or esterase activity, by treatment of a starting solution, which contains the normal amounts of lipase and/or esterase, with 5 ml of sodium hypochlorite solution of the beforementioned activity per liter of microbial rennin containing medium at a pH of about 2.5.

It will be appreciated that the surprisingly obtained results of the rather simple process of the present invention could in no way be predicted by people skilled in the art, as it has appeared that not all conventional oxidizing agents could be used. E.g. hydrogen peroxide did not give any practical result.

As indicated hereinbefore, the process of the present invention may be as well applied to microbial rennin containing liquids, which are exhibiting lipase and/or esterase activity, as to those ones, the lipase and/or esterase activity of which has previously been substantially eliminated  during a recovery process as described in U.S. patent 3,616,233, all relevant parts of which have to be considered as integrally inserted herein.

The microbial rennin activity is determined by the following analytical method.

The time of clotting a defined milk substrate is used to calculate the coagulating activity of the microbial rennin.

One soxhlet unit is the amount of rennin which is clotting one ml of milk substrate in 40 minutes at 35°C.

The substrate is prepared by dissolving 100 g of skimmed powdered milk in one liter of 0.2 g/l calcium chloride solution.

The measured time must be in the range of from 4 to 20 minutes.

The activity is calculated according to the following equation:

$$\frac{40 \times Vo}{t \times cxV} = \text{activity in soxhlet units}$$

Vo = volume of milk substrate

V  = volume of enzymatic solution

t  = clotting time in seconds

c  = concentration of enzyme in V ml


For the _Mucor miehei_ microbial rennin the same analysis is also realized at 63°C.

Due to its thermostability, the activity of _Mucor miehei_ rennin at 63°C is about 170 percent of the activity at 35°C.

The evolution of the activity at 63°C is a direct measurement of the thermostability of the _Mucor miehei_ rennin.

The esterase activity preferably is measured by a test on tributyrin, whereas the lipase activity preferably is measured by a test on an olive oil substrate.

These tests can be carried out according to the methods as described in column 2, lines 45-68, columns 3 and 4 and column 5, lines 1-41 of U.S. patent no. 4065580, the contents of which have to be considered as integrally inserted herein.

The following examples illustrates the practice of the invention and are not in any way to be considered as limiting to the spirit or scope of the invention.

## Example 1

One liter of a microbial rennin containing filtrate was obtained after fermentation with an isolate from Mucor miehei NRRL-A 13042 and recovery of the fermentation broth in the usual way, during which possibly present lipase and/or esterase activity is eliminated according to the method of U.S. patent no. 3,616,233.

This filtrate has an activity of 112 00 Soxhlet units/ml at 35$^O$C and of 193 00 units at 63$^O$C. To this filtrate 10 ml of a sodium hypochlorite stock solution, having an activity corresponding with 150 g active chlorine per liter, were added under stirring in an open vessel.

The temperature of the mixture was kept at 7.5$^O$C and the pH was 4.1.

After 4 hours processing, the activity of the microbial rennin was measured again. The respective activities are listed below.

### Clotting activity in Soxhlet units

|  | 35$^O$C | | 63$^O$C | |
|---|---|---|---|---|
|  | units/ml | percent | units/ml | percent |
| reference sample | 11 200 | 100 | 19 300 | 173 |
| processed sample | 10 400 | 93 | less than 100 | 0 |

## Example 2

To one liter of a microbial rennin containing solution, obtained by filtration of the fermentation broth from a Mucor miehei fermentation as produced according to example 1, followed by ultrafiltration and deactivation of lipase and/or esterase according to the method described in U.S. patent no. 3,616,233, 10 ml of a sodium hypochlorite solution, having an activity corresponding with 150 g/liter of active chlorine, were added under stirring and the liquid was processed in the same way as in example 1.

The clotting activities of the liquid before and after the treatment are listed below.

<u>Clotting activity in Soxhlet units</u>

|  | 35°C | | 63°C | |
|---|---|---|---|---|
|  | units/ml | percent | units/ml | percent |
| reference sample | 224 000 | 100 | 400 000 | 180 |
| processed sample | 216 000 | 96 | less than 200 | 0 |

## Example 3

A filtered microbial rennin solution obtained from a <u>Mucor miehei</u> fermentation as produced according to example 1, is concentrated by ultrafiltration, while the esterase activity is not removed.

To one liter of such solution, 12 ml of sodium hypochlorite (having an activity corresponding with 153 g of active chlorine/liter) were added under stirring at a temperature of 5°C and at pH 2.5. Stirring was continued during 4 hours and the clotting activity and the esterase activity were determined, as listed below.

<u>Clotting activity in Soxhlet units</u>

|  | 35°C | | 63°C | | esterase activity |
|---|---|---|---|---|---|
|  | units/ml | percent | units/ml | percent | ea units |
| reference sample | 250 000 | 100 | 425 000 | 170 | 15 ea |
| processed sample | 237 500 | 95 | less than 200 | 0 | 0.2 ea |

From these values it can clearly be derived that the thermostability of microbial rennin has decreased while simultaneously esterase has been inactivated.

The microbial rennin obtained can be used to make cheddar cheese without developing any rancidity on aging.

The whey resulting from manufacturing cheese does not demonstrate any residual milk clotting activity when normally processed, i.e. when subjected to pasteurisation at 70°C and spray drying.

## Example 4

One liter of a microbial rennin containing filtrate was obtained after fermentation with an isolate from Mucor miehei NRRL A 13042 and the recovery of the fermentation broth in the usual way, during which possibly present lipase and/or esterase activity is eliminated according to the method of the U.S. patent 3,616,233.

This filtrate has an activity of 14.500 soxhlet units/ml at 35°C and 25.400 soxhlet units at 63°C. The lipase activity is 8 units/ml. This filtrate is acidified to pH 3.0 and mixed with 0.25 g of $NaClO_2$ per liter of microbial rennin containing liquid. The temperature is 20°C and the mixture is kept during 4 hours at this temperature. The activities were measured again, and listed below.

#### Clotting activity in Soxhlet units

| | 35°C | | 63°C | | esterase activity |
| | units/ml | percent | units/ml | percent | ea units |
|---|---|---|---|---|---|
| reference sample | 14 500 | 100 | 25 400 | 175 | 8 |
| processed sample | 13 750 | 95 | 250 | 1 | 0.26 |

If the same test is run at pH 4.0 using 1 g/l of $NaClO_2$ under the same conditions of temperature and time, the following results are obtained:

#### Clotting activity in Soxhlet units

| | 35°C | | 63°C | | esterase activity |
| | units/ml | percent | units/ml | percent | ea units |
|---|---|---|---|---|---|
| reference sample | 14 500 | 100 | 25 000 | 172 | 8 |
| processed sample | 14 050 | 97 | 375 | 1.5 | 0.224 |

0028044

Claims:

1. A thermally sensitive microbial rennin, which has retained the major part of its milk coagulating activities at temperatures below 60°C.

2. A thermally sensitive microbial rennin, as claimed in claim 1, which is substantially free of lipase and/or esterase activity.

3. A thermally sensitive microbial rennin, which will loose substantially all of its milk coagulating activity at temperatures above 60°C.

4. A process for the preparation of a thermally sensitive microbial rennin, which comprises treatment of an aqueous microbial rennet containing liquid with an agent selected from the group consisting of the sodium, potassium and calcium salts of per acetic acid, periodic acid, hypochloric acid, chloric acid and iodic acid and mixtures thereof.

5. A process as claimed in claim 4, in which said agent is sodium hypochlorite.

6. A process as claimed in claim 5, in which said agent is present in an activity, corresponding with 0.5-4 g of active chlorine per liter of microbial rennin containing medium, obtained by filtration of the fermentation broth.

7. A process as claimed in claim 6, in which said oxidizing agent is present in an activity, corresponding with 1.5-2 g of active chlorine per liter of microbial rennin containing medium.

8. A process as claimed in claim 4, in which the temperature is in the range of from -10°C to +25°C.

9. A process as claimed in claim 4, in which the pH is selected in the range from 2 to 8.

10. A process as claimed in claim 9, in which the pH is selected in the range from 2.5 to 5.

11. A thermally sensitive microbial rennin, which has retained the major part of its milk coagulating activities at temperatures below 60°C, as obtained by the process of claims 3-10.

12. A thermally sensitive microbial rennin, according to claim 11, which is substantially free of lipase and/

0028044

or esterase activity.

13. A thermally sensitive microbial rennin, which will loose substantially all of its milk coagulating activity above 60°C, as obtained by the process of claims 3-10.

14. A process for the preparation of cheese, characterized in that a thermally sensitive microbial rennin of claim 1 is used.

15. A process for the preparation of food stuffs characterized in that whey, resulting from the process of claim 14, and milk or a liquid derived from milk, are used.

16. Cheese, obtained according to the process of claim 14.

17. Food stuffs, essentially derived from whey, resulting from the process of claim 14.

0028044

Claims for Austria:

1. A process for the preparation of a thermally sensitive microbial rennin, which comprises treatment of an aqueous microbial rennet containing liquid with an agent selected from the group consisting of the sodium, potassium and calcium salts of per acetic acid, periodic acid, hypochloric acid, chloric acid and iodic acid and mixtures thereof.

2. A process as claimed in claim 1, in which said agent is sodium hypochlorite.

3. A process as claimed in claim 2, in which said agent is present in an activity, corresponding with 0.5-4 g of active chlorine per liter of microbial rennin containing medium, obtained by filtration of the fermentation broth.

4. A process as claimed in claim 3, in which said oxidizing agent is present in an activity, corresponding with 1.5-2 g of active chlorine per liter of microbial rennin containing medium.

5. A process af claimed in claim 1, in which the temperature is in the range of from -10°C to +25°C.

6. A process as claimed in claim 1, in which the pH is selected in the range from 2 to 8.

7. A process as claimed in claim 6, in which the pH is selected in the range from 2.5 to 5.

8. A thermally sensitive microbial rennin, which has retained the major part of its milk coagulating activities at temperatures of below 60° C, as obtained by the process of claims 1-7.

9. A thermally sensitive microbial rennin, according to claim 8, which is substantially free of lipase and/ or esterase activity.

10. A thermally sensitive microbial rennin, which will loose substantially all of its milk coagulating activity abo ve 60°C, as obtained by the process of claims 1-7.

11. A process for the preparation of cheese, characterized in that a thermally sensitive microbial rennin of claim 1 is used.

12. A process for the preparation of food stuffs characterized in that whey, resulting from the process of

0028044

claim 11, and milk or a liquid derived from milk, are used.

13. Cheese, obtained according to the process of claim 11.

14. Food stuffs, essentially derived from whey, resulting from the process of claim 11.

# EUROPEAN SEARCH REPORT

## DOCUMENTS CONSIDERED TO BE RELEVANT

| Category | Citation of document with indication, where appropriate, of relevant passages | Relevant to claim |
|---|---|---|
| X | FR - A - 1 401 474 (CHAS PFIZER & CO.) <br><br> * Page 2, column 1, lines 47-48; example 1; abstract * <br><br> & US - A - 3 275 453 <br><br> & US - A - 3 212 905 <br><br> -- | 1,3, 11,13, 17 |
| X | JOURNAL OF DAIRY SCIENCE, vol. 57, no. 5, 1974, page 591, M52 G.A. SOMKUTI et al.: "Milk clotting protease from psychrophilic fungi" <br><br> * Whole article * <br><br> -- | 1,3, 11,13, 17 |
| X | CHEMICAL ABSTRACTS, vol. 76, 1972, page 272, no. 71083j Columbus, Ohio, U.S.A. YUAN-CHI SU et al.: "Milk-clotting enzymes from microorganisms. II. Purification and physicochemical properties of the enzymes and their application in cheese manufacture" <br><br> & CHUNG KUO NUNG YEH HUA HSUEH HUI CHIH 1971, 9(1-2), 80-91 <br><br> * Abstract * <br><br> -- | 1,3, 11,13, 17 |
| X | CHEMICAL ABSTRACTS, vol. 87, 1977, page 524, no. 132475q Columbus, Ohio, U.S.A. CHIN-WEN LIN et al.: "Studies on the manufacture of oriental type cheese -The proteolytic properties of Mucor javanicus" <br><br> & CHUNG-KUO NUNG YEH HUA HSUEH HUI CHIH 1977, 15(1-2), 49-58 <br><br> * Abstract * <br><br> -- ./. | 1,3, 11,13, 17 |

**CLASSIFICATION OF THE APPLICATION (Int. Cl.³)**

C 12 N 9/58
9/99
A 23 C 19/032
21/00

**TECHNICAL FIELDS SEARCHED (Int. Cl.³)**

C 12 N 9/00
9/48
9/50
9/58
A 23 C 19/032

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| The present search report has been drawn up for all claims | | |
|---|---|---|
| Place of search <br> The Hague | Date of completion of the search <br> 30-01-1981 | Examiner <br> DESCAMPS |

EPO Form 1503.1 06.78

European Patent
Office

**EUROPEAN SEARCH REPORT**

EP 80 20 0981
-2-

| | DOCUMENTS CONSIDERED TO BE RELEVANT | | CLASSIFICATION OF THE APPLICATION (Int. Cl. 3) |
|---|---|---|---|
| Category | Citation of document with indication, where appropriate, of relevant passages | Relevant to claim | |
| | <u>DE - A - 2 300 474</u> (SOCIETE DES PRO-DUITS NESTLE) <br><br> * Page 10, lines 8-10 * <br><br> & GB - A - 1 372 800 <br><br> -- <br><br> CHEMICAL ABSTRACTS, vol. 89, 1978, page 429, no. 161696j Columbus, Ohio, U.S.A. K.N. PEARCE: "The inactivation of rennet by chlorinated water" <br><br> & N.Z.J. DAIRY SCI. TECHNOL. 1978, 13(2), 127-8 <br><br> * Abstract * <br><br> -- | 1,3, 11,13-17 | |
| D | BIOCHIMICA ET BIOPHYSICA ACTA, vol. 328, 1973, pages 52-60 P.A. McBRIDE-WARREN et al.: "Structural and functional determinants of mucor miehei protease. II. Circular dichroic studies on the native and periodate-oxidized enzyme" <br><br> * Page 56, paragraph 2; table 1 * <br><br> -- | 1,4,11 | |
| P,E | <u>FR - A - 2 426 696</u> (MILES LAB.) <br><br> * Examples 1-7; claims 1,2,8-10 * <br><br> & GB - A - 2 024 828 <br><br> -- | 1,3, 11,13-17 | |
| P,E | <u>FR - A - 2 445 372</u> (NOVO INDUSTRI) <br><br> * Examples 1-7; claims 1,8,9 * <br><br> & GB - A - 2 038 339 <br><br> -- <br><br> ./. | 1,3, 11,13-17 | |

TECHNICAL FIELDS SEARCHED (Int. Cl. 3)

EPO Form 1503.2 06.78

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| E | <u>GB - A - 2 045 773</u> (NOVO INDUSTRI)<br>* Whole document * | 1,3,4<br>8-11,<br>13-17 | |
| E | <u>GB - A - 2 045 772</u> (NOVO INDUSTRI)<br>* Whole document * | 1-17 | |
| E | <u>WO - A - 80/02225</u> (CHR. HANSEN'S LAB.)<br>* Page 3, line 35 - page 4, line 5; claims 1,5-7,10,20-26 * | 1-4,8-<br>17 | |
| | <u>FR - A - 793 098</u> (AKTIESELSKABET DANSK GAERINGS INDUSTRI)<br>* Page 1, line 47 - page 2, line 35; example 3 * | 4 | |

**TECHNICAL FIELDS SEARCHED (Int. Cl. 3)**